# FASCICULE DE BREVET EUROPEEN

(11) **EP 0 743 050 B1**
(45) Date de publication et mention de la délivrance du brevet: **18.06.2003**
(21) Numéro de dépôt: 96400707.4
(22) Date de dépôt: 02.04.1996
(51) Int. Cl.: A61F 2/34

(54) **Cotyle pour prothèse de hanche**
Gelenkpfanne für Hüftprothese
Acetabular cup for hip proshesis

(30) Priorité: 27.04.1995 FR 9505059
(43) Date de publication de la demande: 20.11.1996
(73) Titulaire: Howmedica France, 69351 Lyon Cedex 08 (FR); S.A. BENOIST GIRARD & CIE, F-14200 Herouville St Clair (FR); Alexandre, Gérard, 78670 Villennes S/Seine (FR); Feron, Jean-Marc, 94300 Vincennes (FR); Lesaux, Daniel, 29219 Le Relerq-Kerhuon (FR); Musset, Thierry, 56260 Larmor Plage (FR); Tomeno, Bernard, 94150 Rungis (FR); Veinberg, Jean, 77300 Fontainebleau (FR); Vielpeau, Claude, 14200 Herouville-Saint-Clair (FR)
(72) Inventeur: Alexandre, Gérard, 78670 Villennes S/Seine (FR); Feron, Jean-Marc, 94300 Vincennes (FR); Lesaux, Daniel, 29219 Le Relerq-Kerhuon (FR); Musset, Thierry, 56260 Larmor Plage (FR); Tomeno, Bernard, 94150 Rungis (FR); Veinberg, Jean, 77300 Fontainebleau (FR); Vielpeau, Claude, 14200 Herouville Saint Clair (FR); Verleyen, Jean-Marie, 69520 Grigny (FR); Baron, Lionel, 14150 Ouistreham (FR)
(74) Mandataire: Santarelli

(56) Documents cités:
- EP-A- 0 381 351
- DE-A- 3 310 944
- DE-A- 3 602 081
- DE-A- 4 239 263
- DE-U- 9 312 150
- FR-A- 2 413 078

## Description

La présente invention a pour objet un cotyle pour prothèse de hanche.

Les prothèses de hanche classiques comportent un élément fémoral et un élément appelé cotyle ; le cotyle est en général en forme d'hémisphère creuse, qui coopère avec la tête sphérique de l'élément fémoral pour remplacer l'articulation naturelle de la hanche. Eventuellement, une pièce intermédiaire en matière plastique est insérée entre l'élément fémoral et le cotyle, qui sont le plus souvent en matière métallique, pour éviter les frictions et faciliter les glissements.

Les cotyles conventionnels doivent être mis en place dans la cavité coxo-fémorale du patient par des moyens de fixation mécanique qui soit causent un traumatisme osseux, dû par exemple à des déchirures provoquées par des vis de fixation, soit nécessitent des perçages des parois du cotyle, qui génèrent des frictions et des blocages par production de débris métalliques, soit les deux en même temps.

La demande de brevet allemand No. 3602081 décrit un cotyle dont la fixation est réalisée au moyen d'un filetage disposé sur la surface externe de ce cotyle.

La demande de brevet Français No. 2413078 décrit une prothèse de hanche destinée au traitement chirurgical des malfonctions atteignant l'articulation de la hanche en transmettant le mieux possible les pressions entre prothèse et cotyle, et en diminuant au maximum les frottement entre prothèse et cotyle.

La présente invention a pour objet de surmonter ces inconvénients.

Elle consiste en un cotyle pour prothèse de hanche en forme de calotte sphérique, la paroi externe du cotyle étant sphérique et le centre de la sphère définissant cette paroi externe se trouvant sur l'axe de révolution du cotyle au-delà du plan défini par l'extrémité ouverte du cotyle par rapport au pôle de la calotte, ledit cotyle étant apte à l'insertion à force dans une cavité osseuse et présente un revêtement externe d'hydroxy-apatite.

Dans une mise en oeuvre préférée, les parois interne et externe du cotyle sont sphériques, la sphère définissant la paroi externe ayant un rayon supérieur à celui de la sphère définissant la paroi interne et le centre de cette dernière sphère correspondant au centre de l'extrémité circulaire ouverte du cotyle.

Un tel cotyle présente l'avantage de pouvoir rentrer à force dans une cavité osseuse coxo-fémorale dont le diamètre correspond à la hauteur du cotyle ; la surépaisseur, située à l'extrémité équatoriale ouverte du cotyle bloque ce dernier sur place, par compression de l'os sur toute la périphérie de la cavité coxo-fémorale. Le revêtement externe d'hydroxy-apatite ou de péri-apatite™ a l'effet d'un dispositif d'ancrage.

Pour éviter une rotation intempestive du cotyle dans la cavité, on peut prévoir, sur sa paroi externe, des éléments anti-rotation comme des ailerons périphériques.

L'ancrage du cotyle dans la cavité peut également être facilité par la présence, sur la paroi externe du cotyle, d'un jeu de rainures circulaires situées dans des plans parallèles au plan de son extrémité ouverte et intermédiaires entre ce plan et le pôle de la calotte sphérique.

Ce jeu de rainures dans des plans correspondants à des latitudes peut être complété par des rainures allant du pôle vers l'extrémité ouverte du cotyle et situées dans des plans correspondant à des longitudes.

Le cotyle, qui est en alliage de titane ou en d'autres matériaux, peut être garni à sa partie périphérique interne d'une couronne crantée et/ou d'un système de blocage, permettant l'insertion et le maintien d'un insert intermédiaire en matière plastique à bas coefficient de friction, tel qu'une polyoléfine, ou en matière céramique.

L'invention va maintenant être illustrée à l'aide des dessins annexés dans lesquels :
- la figure 1 représente une vue en coupe selon la ligne I I de la figure 3 d'une mise en oeuvre préférée de l'invention,
- la figure 2 représente une vue de face du cotyle représenté sur la figure 1,
- la figure 3 une vue de dessus et la figure 4 une vue de dessous du cotyle représenté sur les figures précédentes.

Le cotyle selon l'invention est représenté sur les figures avec la référence (1). Il est en forme de calotte sphérique définie par une paroi externe (2) sphérique et une paroi interne (3) sphérique et présente une extrémité ouverte circulaire (4).

Dans la mise en oeuvre représentée sur les figures, le centre de la sphère définissant la paroi interne (3) se trouve au centre de l'extrémité ouverte circulaire (4).

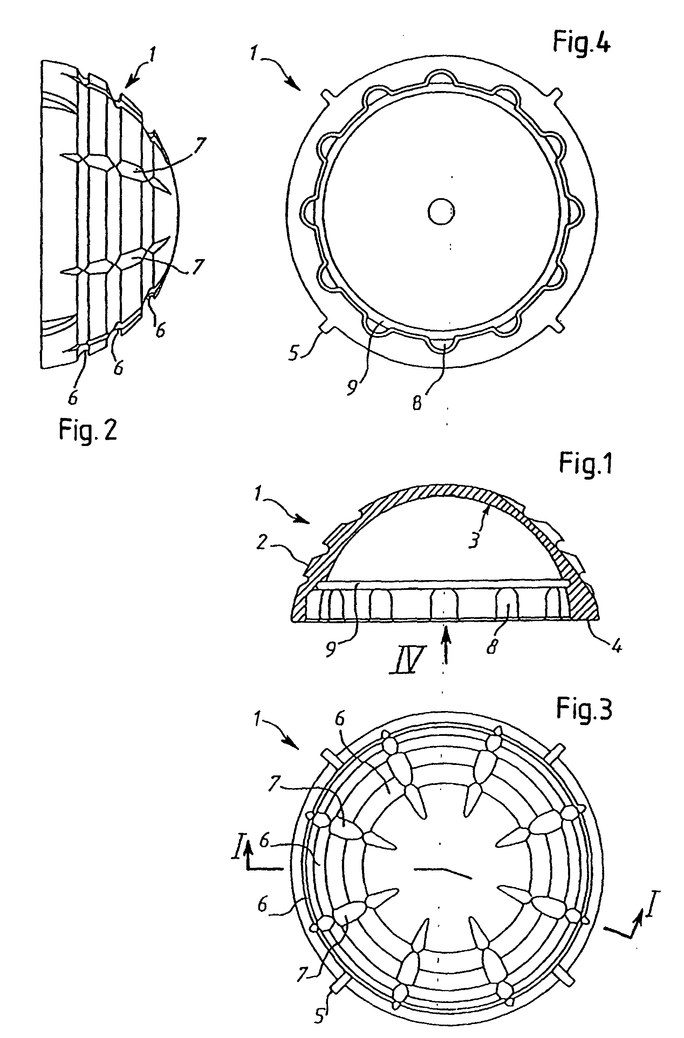

Par contre le centre de la sphère définissant la paroi externe (2) se trouve sur l'axe de révolution du cotyle, au-delà du plan défini par l'extrémité (4), donc en dessous de ce plan, dans la vue représentée sur la figure 1.

Comme le montre clairement cette figure, il en résulte une paroi du cotyle d'épaisseur minimale au pôle et maximale à sa périphérie correspondant à l'extrémité ouverte (4).

La paroi externe (2) du cotyle est munie, à sa partie périphérique inférieure, proche de l'extrémité (4), d'ailerons (5), destinés à s'incruster dans la cavité de l'os iliaque et à prévenir une rotation intempestive du cotyle (voir figure 2).

Un jeu de rainures circulaires (6), dans des plans parallèles au plan de l'extrémité ouverte (4), est creusé à intervalles réguliers entre le pôle et l'extrémité équatoriale du cotyle, sur la paroi externe (2) de ce dessin.

Des rainures (7) longitudinales sont également creusées dans la paroi externe (2) et s'étendent du pôle en direction de l'équateur dans des plans correspondant à des longitudes (voir les figures 2 et 3).

Enfin l'extrémité ouverte (4) du cotyle est munie, à sa partie périphérique interne d'une couronne crantée (8) et de gorges d'encliquetage (9), destinées à la réception et au blocage d'un insert en matière plastique ou céramique, destiné à être interposé entre le cotyle métallique et la tête sphérique de l'élément fémoral de la prothèse (voir figure 4).

Un tel cotyle peut être inséré à force dans une cavité dont le diamètre correspond à la hauteur totale du cotyle, mesurée sur l'axe de révolution à partir du pôle de sa paroi externe jusqu'au centre de son extrémité inférieure ouverte.

Il y est maintenu sous pression par l'élément fémoral de la prothèse ; il ne peut pas tourner, grâce aux éléments anti-rotation (5) et son système d'ancrage à rainures (6) et (7), complété éventuellement par un revêtement externe d'hydroxy-apatite assure sa compatibilité avec la cavité coxo-fémorale dans l'os iliaque du patient.

## Revendications

1. Cotyle pour prothèse de hanche en forme de calotte sphérique, la paroi externe (2) du cotyle étant sphérique et le centre de la sphère définissant cette paroi externe se trouvant sur l'axe de révolution du cotyle au delà du plan défini par l'extrémité ouverte (4) du cotyle par rapport au pôle de la calotte, ledit cotyle étant apte à l'insertion à force dans une cavité osseuse et présente un revêtement externe d'hydroxy-apatite.

2. Cotyle selon la revendication 1 **caractérisé en ce que** les parois interne (3) et externe (2) du cotyle sont sphériques, la sphère définissant la paroi externe (2) ayant un rayon supérieur à celui de la sphère définissant la paroi interne (3) et le centre de cette dernière sphère correspondant au centre de l'extrémité circulaire ouverte (4) du cotyle.

3. Cotyle selon l'une quelconque des revendications précédentes, **caractérisé en ce qu'**il présente, sur sa paroi externe (2), des éléments anti-rotation, notamment des ailerons périphériques (5).

4. Cotyle selon l'une quelconque des revendications précédentes, **caractérisé en ce qu'**il présente sur sa paroi externe (2), un jeu de rainures circulaires (6) situées dans des plans parallèles à son extrémité ouverte (4) et intermédiaires entre le pôle et l'extrémité ouverte (4) du cotyle.

5. Cotyle selon l'une quelconque des revendications précédentes, **caractérisé en ce qu'**il présente sur sa paroi externe (2), un jeu de rainures longitudinales (7) s'étendant du pôle vers l'extrémité ouverte (4) dans des plans correspondant à des longitudes.

6. Cotyle selon l'une quelconque des revendications précédentes, **caractérisé en ce qu'**il présente à sa partie périphérique interne proche de l'extrémité ouverte (4), une couronne crantée (8) et éventuellement des gorges d'encliquetage (9) pour la réception et le blocage d'un insert en matière plastique ou céramique.

## Patentansprüche

1. Gelenkpfanne für eine Hüftprothese, in Form einer sphärischen Kalotte, wobei die Außenwand (2) der Gelenkpfanne sphärisch ausgebildet und der Mittelpunkt der die Außenwand definierenden Kugel auf der Schwenkachse der Gelenkpfanne in der durch das offene Ende (4) der Gelenkpfanne bezüglich des Poles der Kalotte definierten Ebene angeordnet ist, und wobei die Gelenkpfanne zum Einsetzen unter Krafteinwirkung in eine Vertiefung am Knochen ausgelegt ist und eine Außenbeschichtung aus Hydroxyapatit aufweist.

2. Gelenkpfanne nach Anspruch 1, **dadurch gekennzeichnet, dass** die Innen- (3) und Außenwände (2) der Gelenkpfanne sphärisch ausgebildet sind, wobei die die Außenwand (2) definierende Kugel einen Radius hat, der größer ist als der der die Innenwand (3) definierenden Kugel, und der Mittelpunkt der letztgenannten Kugel dem Mittelpunkt des runden, offenen Endes (4) der Gelenkpfanne entspricht.

3. Gelenkpfanne nach einem der vorhergehenden Ansprüche, **dadurch gekennzeichnet, dass** sie auf ihrer Außenwand (2) Elemente zur Drehsicherung, insbesondere Umfangsflügel (5), aufweist.

4. Gelenkpfanne nach einem der vorhergehenden Ansprüche, **dadurch gekennzeichnet, dass** sie auf ihrer Außenwand (2) eine Gruppe von Ringnuten (6) aufweist, welche in zu ihrem offenen Ende (4) parallelen Ebenen, zwischen dem Pol und dem offenen Ende (4) angeordnet sind.

5. Gelenkpfanne nach einem der vorhergehenden Ansprüche, **dadurch gekennzeichnet, dass** sie auf ihrer Außenwand (2) eine Gruppe von Längsnuten (7) aufweist, welche sich vom Pol zum offenen Ende (4) hin in Ebenen erstrecken, die mit den Längen übereinstimmen.

6. Gelenkpfanne nach einem der vorhergehenden Ansprüche, **dadurch gekennzeichnet, dass** sie an ihrem Innenumfangsabschnitt nahe dem offenen Ende (4) einen verzahnten Kranz (8) sowie gegebenenfalls Rastvertiefungen (9) für die Aufnahme und Blockierung eines Einsatzes aus einem Kunststoff- oder Keramikmaterial aufweist.

## Claims

1. An acetabulum for a hip prosthesis in the form of a spherical cap, the outer wall (2) of the acetabulum being spherical and the center of the sphere defining that outer wall being located on the axis of revolution of the acetabulum beyond the plane defined by the open end (4) of the acetabulum with respect to the pole of the cap, said acetabulum being adapted for force-fitting in a bony cavity and has an outer hydroxyapatite coating.

2. An acetabulum according to claim 1, **characterized in that** the inner (3) and outer (2) walls of the acetabulum are spherical, the sphere defining the outer wall (2) having a radius greater than that of the sphere defining the inner wall (3) and the center of the latter sphere corresponding to the center of the open circular end (4) of the acetabulum.

3. An acetabulum according to any one of the preceding claims, **characterized in that** it has, on its outer wall (2), anti-rotation members, in particular peripheral fins (5).

4. An acetabulum according to any one of the preceding claims, **characterized in that** it has on its outer wall (2), a set of circular grooves (6) situated in planes parallel to its open end (4) and intermediate between the pole and the open end (4) of the acetabulum.

5. An acetabulum according to any one of the preceding claims, **characterized in that** it has on its outer wall (2), a set of longitudinal grooves (7) extending from the pole towards the open end (4) in planes corresponding to lines of longitude.

6. An acetabulum according to any one of the preceding claims, **characterized in that** it has on its inner peripheral portion, close to the open end (4), a notched annulus (8) and possibly channels (9) for snap-fitting for receiving and fixing an insert of plastics or ceramic material.
